(19) Europäisches Patentamt
European Patent Office
Office européen des brevets

(11) EP 4 663 191 A1

(12) EUROPEAN PATENT APPLICATION
published in accordance with Art. 153(4) EPC

(43) Date of publication:
17.12.2025 Bulletin 2025/51

(21) Application number: 24752887.0

(22) Date of filing: 07.02.2024

(51) International Patent Classification (IPC):
*A61K 31/496* (2006.01) *A61K 31/4439* (2006.01)
*A61K 31/404* (2006.01) *C07D 403/06* (2006.01)
*C07D 401/14* (2006.01) *A61P 35/00* (2006.01)

(52) Cooperative Patent Classification (CPC):
**A61K 31/404; A61K 31/4439; A61K 31/496; A61P 35/00; C07D 401/14; C07D 403/06**

(86) International application number:
PCT/CN2024/076530

(87) International publication number:
WO 2024/165032 (15.08.2024 Gazette 2024/33)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 08.02.2023 CN 202310100761

(71) Applicants:
- **Qilu Animal Health Products Co., Ltd.**
  **Jinan, Shandong 250105 (CN)**
- **QILU PHARMACEUTICAL CO., LTD.**
  **Jinan, Shandong 250100 (CN)**

(72) Inventors:
- **KONG, Mei**
  **Jinan, Shandong 250105 (CN)**
- **MA, Qingzhu**
  **Jinan, Shandong 250105 (CN)**
- **WU, Lianyong**
  **Jinan, Shandong 250105 (CN)**
- **ZHENG, Li**
  **Jinan, Shandong 250105 (CN)**
- **FENG, Yanyan**
  **Jinan, Shandong 250105 (CN)**
- **LIU, Quancai**
  **Jinan, Shandong 250105 (CN)**
- **ZHENG, Huanqing**
  **Jinan, Shandong 250105 (CN)**

(74) Representative: **Müller Schupfner & Partner Patent- und Rechtsanwaltspartnerschaft mbB (Muc)**
**Bavariaring 11**
**80336 München (DE)**

# (54) USE OF TYROSINE KINASE INHIBITOR

(57) Provided in the present application are the use of a compound represented by formula (I) or pharmaceutically acceptable salts thereof in the preparation of drugs used for treating mast cell tumors in non-human mammals, and a method of using same to treat mast cell tumors in non-human mammals.

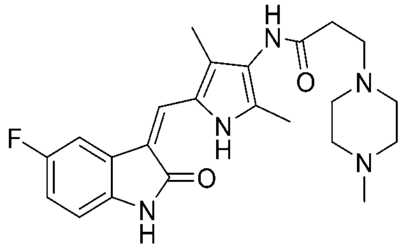

Formula (I)

EP 4 663 191 A1

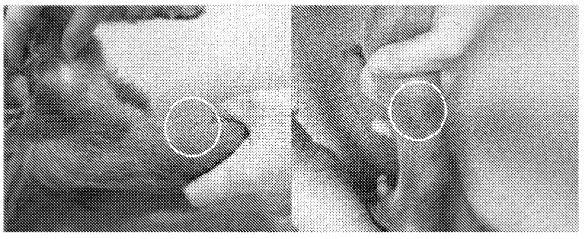

FIG. 3

## Description

The present application claims the priority of the Chinese Patent Application NO. 202310100761.3, titled "USE OF TYROSINE KINASE INHIBITOR", filed before the China National Intellectual Property Administration on February 8, 2023, which is incorporated herein by reference in its entirety.

## Technical Field

The present application relates to the field of pharmaceutical and chemical engineering, and specifically relates to a use of a compound represented by formula (I) or a pharmaceutically acceptable salt thereof in the treatment of mast cell tumors.

## Background

With the development of society, more and more people keep pets. According to the research data from the "2021 Pet Industry White Paper", the number of pet cats and dogs in Chinese urban households is 58.06 million and 54.29 million, respectively. The numbers of aging pets and age-related diseases of pets are increasing over time. Especially, the incidence of pet tumors has increased significantly, among which malignant tumors directly threaten the life and health of pets.

Animal skin tumor is one of the most common tumors. According to the survey, the incidence of canine skin tumors is about 1/3 of that of canine tumors, and malignant tumors account for 20-40% of skin tumors. The occurrence of skin tumors has a significant impact on the health of pet dogs. Wherein, mast cell tumor (MCT) is the most common skin tumor in dogs and the second among skin tumors in cats. Mast cell tumor occurs in dogs of all ages, but it is more common in elderly dogs. There is no gender predisposition to the disease. Among dogs, the disease is common for crossbreed dogs, whereas the disease is highly prevalent in certain breeds such as Boxer, Labrador, Pug, Beagle, and the like. Mast cell tumors are frequently occurring in the mesenchymal tissues of the skin of dogs, and have the appearance of alopecia, reddened skin nodules that can occur anywhere on the body surface.

The neoplastic hyperplasia of mast cells is known as mast cell tumors (MCTs). Mast cell tumors are characterized by abnormal proliferation and accumulation of mast cells. Neoplastic mast cell clusters can spontaneously degranulate, releasing biologically active molecules that can exert severe and potentially lethal effects, such as allergic reactions, gastric and duodenal ulcer or perforation, glomerular disease, hemorrhage and the like. In canines, mast cell tumors (MCTs) have extremely diverse clinical manifestations and biological behaviors. In other species, such as felines, there is less variation in MCT biological behavior. In humans, mast cell tumor is called mastocytosis and usually presents as a systemic disease rather than a defined mass. Mastocytosis in humans can range from spontaneously regressing skin lesions to highly aggressive multi-system malignancies.

The incidence of canine mast cell tumor accounts for 16-21% of all skin tumors, with a malignancy rate of 19-39%. The metastasis rate of poorly differentiated mast cell tumor is 55% to 96%. Generally, it first metastasizes to the lymph nodes, then to the spleen and liver, and finally to the bone marrow. At this time, a large number of mast cells can be found in the peripheral blood, which is called mastocythemia. Canines with a malignant mast cell tumor have unsatisfactory survival periods and quality of life. With the gradual application of molecular research methods in the clinical practice of small animals, studies have found that nearly 15% to 40% of canine mast cell tumors contain c-kit gene mutations. The mutations discovered so far include point mutations, internal tandem duplication (ITD) mutations, and base deletion mutations. A significant association between the c-kit mutations and the development and progression of canine mast cell tumors has been well documented. Mast cell tumor includes a cutaneous mast cell tumor and a systemic mast cell tumor. Systemic mast cell tumor refers to a disease involving multiple organs or systems caused by the neoplastic hyperplasia of mast cells, including or excluding the symptoms of the cutaneous mast cell tumor. Examples include visceral MCTs, intestinal MCTs, etc.

The more commonly used system for grading the pathology of canine mast cell tumors is the Patnaik grading system. In the Patnaik grading system, mast cells in grade I MCTs are well differentiated; and neatly scatter among dermal collagen fibers; in which cytoplasmic spaces are ample and filled with granules; nuclear chromatin are condensed; and there is no visible nucleolus or a mitotic cell. Mast cells in grade II MCTs are well differentiated; with cytoplasmic spaces being ample and filled with granules; mild cell atypia; some porous nuclei with pronounced nucleoli, some of which are herpetic; and a moderate number of mitotic cells. Mast cells in grade III MCTs have blurred cytoplasm; moderate to severe atypia; deeply stain nuclei with pronounced nucleoli; visible mitotic cells.

Unlike canine MCTs, which mostly start in the skin/subcutaneous tissue, feline MCTs have three typical classifications and are partially symptomatic with each other. The three classifications are cutaneous MCTs, spleen/-visceral MCTs and intestinal MCTs, respectively. Their etiologies are still unclear, but there are also obvious c-kit active mutations in cat MCTs.

In terms of treatment, surgery combined with radiotherapy is generally used for dogs with locally solitary mast cell tumors without distal metastases. However, due to the expensive radiotherapy equipment and treatment fees, this treatment is currently less acceptable to the majority of animal hospitals and pet owners. In contrast, chemotherapy is required for dogs with distal metastases or very aggressive mast cell tumors.

Tyrosine kinase is a popular research target of human antitumor drugs. Abnormalities in the target pathway in pets also lead to occurrence of tumors. Therefore, tyrosine kinase inhibitors can theoretically be used for a treatment of pet tumors. Tyrosine kinase inhibitors currently used in the treatment of pet tumors include Toceranib, Masitinib, which is especially more effective for the MCT that has mutations in the c-kit gene. The mechanism of the drug is as follows: through competitive inhibition of ATP binding to the mutated tyrosine kinase receptor, blocking the phosphorylation of the activation center of the tyrosine kinase receptor, thereby inhibiting its activation, and ultimately inhibiting the proliferation of tumor cells.

WO2011153814A1 discloses that N-(5-((Z)-(5-fluoro-2-carbonylindole-3-ylidene)methyl)-2,4-dimethyl-1H-pyrrol-3-yl)-3-(4-methylpiperazin-1-yl)propanamide compound has a potential to be developed into an anti-tumor drug. However, its efficacy in treating tumors in non-human mammals (such as pets) has not yet been confirmed.

## Summary

The present application provides use of a compound of formula (I) or a pharmaceutically acceptable salt thereof in the manufacture of a medicament for a treatment of a mast cell tumor in a non-human mammal.

The details are as follows:

The present application provides use of a compound N-(5-((Z)-(5-fluoro-2-carbonylindole-3-ylidene)methyl)-2,4-dimethyl-1H-pyrrol-3-yl)-3-(4-methylpiperazin-1-yl) propanamide represented by formula (I) or a pharmaceutically acceptable salt thereof in the manufacture of a medicament for treatment of a mast cell tumor in a non-human mammal,

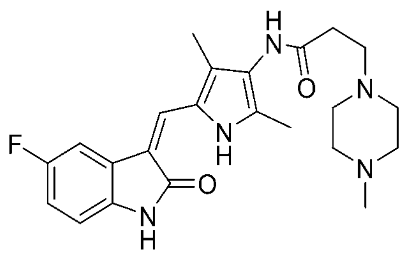

Formula (I).

In some preferred embodiments of the present application, the mast cell tumor described in the above use includes a cutaneous mast cell tumor and a systemic mast cell tumor.

In some preferred embodiments of the present application, the mast cell tumor described in the above use is a cutaneous mast cell tumor.

In some preferred embodiments of the present application, the mast cell tumor described in the above use is a recurrent mast cell tumor.

In some preferred embodiments of the present application, the mast cell tumor described in the above use is an unresectable grade 2 or grade 3 recurrent cutaneous mast cell tumor.

In some preferred embodiments of the present application, the mast cell tumor described in the above use is a tumor caused by a c-kit gene mutation.

In some preferred embodiments of the present application, the compound of formula (I) or the pharmaceutically acceptable salt thereof described in the above use is administered to a non-human mammal at least once daily, or every other day, at a dose of 0.1 mg/kg to 10 mg/kg.

In some preferred embodiments of the present application, the compound of formula (I) or the pharmaceutically acceptable salt thereof described in the above use is administered to a non-human mammal at least once daily, or every other day, at a dose of 1 mg/kg to 5 mg/kg.

In some preferred embodiments of the present application, the compound of formula (I) or the pharmaceutically acceptable salt thereof described in the above use is administered to a non-human mammal at least once daily, or every other day, at a dose of 1.5 mg/kg to 3.5 mg/kg.

In some preferred embodiments of the present application, the compound of formula (I) or the pharmaceutically acceptable salt thereof described in the above use is administered to a non-human mammal at least once daily, or every other day, at a dose of 2 mg/kg to 3.5 mg/kg.

In some preferred embodiments of the present application, the compound of formula (I) or the pharmaceutically

acceptable salt thereof described in the above use is administered at one or more of the following dosages: 2.1 mg/kg, 2.2 mg/kg, 2.3 mg/kg, 2.4 mg/kg, 2.5 mg/kg, 2.6 mg/kg, 2.7 mg/kg, 2.8 mg/kg, 2.9 mg/kg, 3.0 mg/kg, 3.1 mg/kg, 3.2 mg/kg, 3.3 mg/kg, 3.4 mg/kg, and 3.5 mg/kg, at least once daily or every other day to a non-human mammal.

In some preferred embodiments of the present application, the compound of formula (I) or the pharmaceutically acceptable salt described in the above use can be administered to a non-human mammal via any of routes of oral administration, parenteral administration, rectal administration, cutaneous administration, nasal or inhalation administration, intra-tumoral administration, etc.

In some preferred embodiments of the present application, the pharmaceutically acceptable salt of the compound of formula (I) as described in the above use is a dimaleate.

In some preferred embodiments of the present application, the non-human mammal described in the above use is selected from the group consisting of canine, feline, murine and equine.

In another aspect of the present application, it also provides a therapeutic method of a mast cell tumor in a non-human mammal, wherein a therapeutically effective amount of the compound of formula (I) or the pharmaceutically acceptable salt thereof is administered to said non-human mammal:

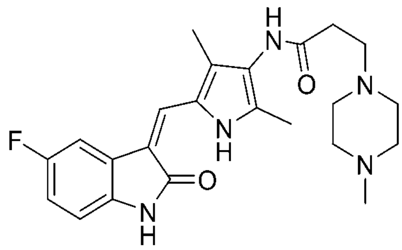

formula (I).

In some preferred embodiments of the present application, the mast cell tumor described in the above therapeutic method includes a cutaneous mast cell tumor and a systemic mast cell tumor.

In some preferred embodiments of the present application, the mast cell tumor described in the above therapeutic method is a cutaneous mast cell tumor.

In some preferred embodiments of the present application, the mast cell tumor described in the above therapeutic method is a recurrent mast cell tumor.

In some preferred embodiments of the present application, the mast cell tumor described in the above therapeutic method is an unresectable grade 2 and grade 3 recurrent cutaneous mast cell tumor.

In some preferred embodiments of the present application, the mast cell tumor described in the above therapeutic method is a tumor caused by a c-kit gene mutation.

In some preferred embodiments of the present application, the compound of formula (I) or the pharmaceutically acceptable salt thereof described in the above-described therapeutic method is administered to a non-human mammal at least once daily, or every other day, at a dose of 0.1 mg/kg to 10 mg/kg.

In some preferred embodiments of the present application, the compound of formula (I) or the pharmaceutically acceptable salt thereof described in the above-described therapeutic method is administered to a non-human mammal at least once daily, or every other day, at a dose of 1 mg/kg to 5 mg/kg.

In some preferred embodiments of the present application, the compound of Formula (I) or the pharmaceutically acceptable salt thereof described in the above-described method of treatment is administered to a non-human mammal at least once daily, or every other day, at a dose of 1.5 mg/kg to 3.5 mg/kg. In some preferred embodiments of the present application, the compound of formula (I) or the pharmaceutically acceptable salt thereof described in the above-described therapeutic method is administered to a non-human mammal at least once daily, or every other day, at a dose of 2 mg/kg to 3.5 mg/kg.

In some preferred embodiments of the present application, the compound of formula (I) or the pharmaceutically acceptable salt thereof described in the above-described therapeutic method is administered at one or more of the following dosages: 2.1 mg/kg, 2.2 mg/kg, 2.3 mg/kg, 2.4mg/kg, 2.5 mg/kg, 2.6 mg/kg, 2.7 mg/kg, 2.8 mg/kg, 2.9 mg/kg, 3.0 mg/kg, 3.1 mg/kg, 3.2mg/kg, 3.3 mg/kg, 3.4 mg/kg, 3.5 mg/kg, at least once daily or every other day to a non-human mammal.

In some preferred embodiments of the present application, the compound of formula (I) or the pharmaceutically acceptable salt described in the above therapeutic method can be administered to a non-human mammal via any of routes of oral administration, parenteral administration, rectal administration, cutaneous administration, nasal or inhalation administration, intra-tumoral administration, etc.

In some preferred embodiments of the present application, the pharmaceutically acceptable salt of the

compound of formula (I) as described in the above therapeutic method is a dimaleate.

In some preferred embodiments of the present application, the non-human mammal described in the above method is selected from the group consisting of canine, feline, murine and equine.

In another aspect of the present application, it also provides the compound represented by formula (I), or the pharmaceutically acceptable salt thereof, which can be used for the treatment of a mast cell tumor in a non-human mammal,

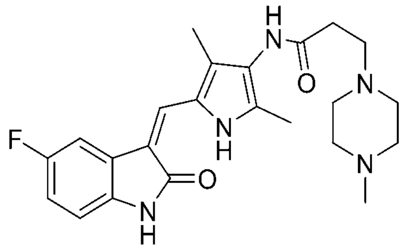

Formula (I).

**Definition and statement**

Unless otherwise indicated, the following terms and phrases used in the present application are intended to have the following meanings. A particular term or phrase should not be considered uncertain or unclear without a specific definition, but should be understood in its ordinary meaning.

The term "pharmaceutically acceptable" refers to those compounds, materials, compositions and/or dosage forms, which are within the range of reliable medical judgment and are suitable for contact with animal tissues without excessive toxicity, irritation, allergic reaction or other problems or complications, and are commensurate with a reasonable benefit/risk ratio.

The term "pharmaceutically acceptable salt" refers to a salt of the compound in the present application, which is prepared by the compound with a specific substituent discovered in the present application and a relatively non-toxic acid or base. When the compound of the present application contains a relatively acidic functional group, a base addition salt can be obtained by contacting a neutral form of such compound with a sufficient amount of a base in a pure solution or a suitable inert solvent. When the compound of the present application contains a relatively basic functional group, an acid addition salt can be obtained by contacting a neutral form of such compound with a sufficient amount of an acid in a pure solution or a suitable inert solvent. The pharmaceutically acceptable salt of the present application can be prepared by a conventional chemical method from a parent compound containing an acid radical or a basic radical. Under normal circumstances, a preparation method of such salts is as follows: in water or organic solvents or a mixture of both, such compounds in forms of free acids or bases react with stoichiometric appropriate bases or acids. Specifically, pharmaceutically acceptable salts are described in e.g. S. M. Berge, et al., J. Pharmaceutical Sciences, 1977, 66:1 in detail. Representative acid addition salts include but are not limited to acetate, adipate, alginate, citrate, aspartate, benzoate, benzenesulfonate, bisulfate, sulfate, butyrate, camphorate, camphor sulfonate, digluconate, glycerophosphate, hemisulphate, heptylate, caproate, fumarate, hydrochloride, hydrobromate, hydriodate, 2-hydroxyl ethanesulfonate (isothiosulfate), lactate, maleate, mesylate, nicotinate, 2-naphthalene sulfonate, oxalate, palmitate, pectate, persulfate, 3-phenylpropionate, picate, pivalate, propionate, succinate, tartrate, thiocyanate, phosphate, malate, glutamate, bicarbonate, p-toluenesulfonate salt, and undecanoate. Similarly, basic nitrogen-containing groups can be quaternized with the following substances: lower alkyl halides, such as chlorides, bromides and iodides of methyl, ethyl, propyl and butyl; dialkyl sulfate esters such as dimethyl sulfate, diethyl sulfate, dibutyl sulfate and dipentyl sulfate; long-chain halides such as chlorides, bromides and iodides of decyl, dodecyl, tetradecyl and octadecyl; aryl alkyl halides such as benzyl bromide and phenethyl bromide, and the like.

The term "therapeutically effective amount" refers to an amount that is sufficient for the compound of the present application or a pharmaceutically acceptable salt thereof to treat a disorder with a reasonable effect/risk ratio suitable for any medical treatment and/or prevention. However, it should be recognized that the total daily dosage of the pharmaceutically acceptable salts and compositions of the compound represented by formula (I) of the present application must be determined by an attending physician within a reliable medical judgment range. For any particular test animal, the specific therapeutically effective dosage level must be determined based on multiple factors, including the disorder being treated and the severity of the disorder; activity of the specific compound used; the specific composition used; age, weight, general health status, gender, and diet of a patient; the specific administration time, administration route, and excretion rate of the compound used; duration of treatment; drugs used in combination or simultaneously with the specific compounds used; and similar factors commonly known in the medical field.

The term "administration" refers to physically introducing a composition comprising a therapeutic agent to a subject using any of a variety of methods and delivery systems known to those skilled in the art. The administration routes include oral administration, parenteral administration, intraperitoneal administration, intravenous administration, arterial administration, transdermal administration, sublingual administration, intramuscular administration, rectal administration, buccal administration, intranasal administration, inhalation, vagina administration, intraocular administration, local administration (such as through a catheter or a stent), subcutaneous administration, intra-adipose administration, intra-articular administration, intraperitoneal administration and intrathecal administration, etc. For certain embodiments of the present application, "administration" refers to delivery into a subject through the oral route.

The term "non-human mammal" used in the present application refers to mammals other than humans at any stage of development. In certain embodiments, said non-human mammal includes, but is not limited to, a rodent (e.g., mouse, rat, rabbit), dog, cat, sheep, cow, horse, primate (e.g., monkey), and/or pig.

The term "grade 2 or grade 3 recurrent cutaneous mast cell tumor" described in the present application refers to a Patnaik Grade II or Grade III recurrent cutaneous mast cell tumor.

Tumor recurrence: generally, it refers to the phenomenon that after a diagnosed tumor is clinically cured by radical treatment (including surgery, radiotherapy, chemotherapy and other treatments), similar tumor appears again in and around the primary site or in other parts of the body in the future. Tumor recurrence includes local recurrence and distant metastasis.

Beneficial effects of the present application:

The compound of formula (I) or a pharmaceutically acceptable salt thereof of the present application significantly inhibits the growth of a mast cell tumor, prolongs the survival of the animal, and reduces types and incidence of adverse reactions in a non-human mammal. The compound of formula (I) or a pharmaceutically acceptable salt thereof of the present application has good efficacy and safety in the treatment of a mast cell tumor in a non-human mammal.

## Brief Description of the Drawings

The accompanying drawings illustrated herein are used to provide a further understanding of the present application and form part of the present application. The schematic examples of the present application and their description are used to explain the present application and do not constitute an undue limitation of the present application.

FIG. 1 shows a photograph of the baseline measurement of the tumor in the Golden Retriever case in Experimental Example 3 on October 3 (2.5×2.2 cm).

FIG. 2 shows a photograph of the efficacy of the compound of formula (I) in the Golden Retriever case in Experimental Example 3 on October 17 (2.2×2.1 cm).

FIG. 3 shows a photograph of the efficacy of the compound of formula (I) in the Golden Retriever case in Experimental Example 3 on November 1 (the mass is shown in the white circle).

## Detailed Description

The application is described in detail below by way of examples, but these examples are for illustrative purposes only and do not limit any scope of the present application. Again, the present application is not limited to any specific preferred embodiments described herein. Those skilled in the art should understand that equivalent substitutions of the technical features of the present application, or corresponding improvements, still fall within the scope of protection of the present application. Unless otherwise indicated, the examples are carried out under conventional experimental conditions.

The compound of formula (I) or a salt thereof of the present application can be prepared and obtained with reference to the methods documented in patents WO2011153814A1, CN104119321A or other methods known in the art.

### Experimental Example 1: The inhibition effect of compound (I) of the present application on the proliferation of a tumor cell

Test sample: the dimaleate of the compound of formula (I) of the present application.

The inhibition effect of the dimaleate of the compound of formula (I) on the growth of mouse mast cell tumor cells P815 cells (ATCC-TIB-64) was detected by MTT assay (thiazolyl blue colorimetric assay), and $IC_{50}$ (half inhibitory concentration) was calculated and obtained.

| Test Sample | P815 ($IC_{50}$, μM) |
|---|---|
| The dimaleate of the compound of formula (I) | 0.474 |

**Experimental Example 2: *in vivo* efficacy evaluation of the compound of formula (I) of the present application on a tumor DBA/2 mouse model of subcutaneous homologous transplantation of mouse mast cell tumor P815 cells**

Test sample: the dimaleate of the compound of formula (I) of the present application (content: 99.7%), and Toceranib phosphate (content: 98.2%, commercially available), the concentration and dosage of administration of the test sample prepared in the following implementation are all calculated in terms of free alkali.

Test animal: the species is mouse; the strain is DBA/2 mouse; the age is 6-8 weeks old, and the weight is 19-24 g; and the gender is female.

Experimental methods and steps: mouse mast cell tumor P815 cells (ATCC-TIB-64) were adherent and cultured *in vitro*. The culture conditions were DMEM medium, 10 vol% fetal bovine serum, 1 vol% double antibody, and cultured in a cell incubator at 37 °C with 5% $CO_2$. Conventional digestion treatment and passage were carried out twice a week with trypsin-EDTA. When the cell saturation reached 80-90% and the quantity satisfied the requirements, the cells were collected, counted and inoculated.

0.1 mL of PBS containing $5\times10^5$ P815 cells was subcutaneously inoculated into the subcutaneous area of the right forelimb axilla of each mouse. At day 8 after cell inoculation, when the average tumor volume reached 100 $mm^3$, group administration was started. The experiment was grouped into a negative control group (without an active ingredient), an administration group, and a positive control group (with the active ingredient Toceranib), with 8 mice in each group. The negative control (vehicle), the dimaleate of the compound of formula (I), and Toceranib phosphate were administered orally once daily for an 8-day administration period. The specific preparation method is shown in Table 1, and the administration protocol is shown in Table 2. Health status and mortality of animals were monitored every day. Routine examinations included observing the effects of tumor growth and drug treatment on daily behavioral performance of animals, such as behavioral activities, food and water intake (only visually), weight changes (measuring weight twice a week), appearance signs or other abnormal conditions.

**Table 1. Preparation methods of the test samples**

| Compound | Dosage of administration (mg/kg) | Methods of preparation | Concentration (mg/mL) | Storage condition |
|---|---|---|---|---|
| Vehicle | -- | 5 vol% polyoxyethylated castor oil + 5 vol% anhydrous ethanol + 90 vol% sterilized water for injection | -- | Prepared and used immediately |
| Dimaleate of the compound of formula (I) | 20 | 7.44 mg of the dimaleate of the compound of formula (I) was weighed into a preparation bottle, and added with about 0.240 mL of a mixed liquid of 5 vol% polyoxyethylene castor oil and 5 vol% anhydrous ethanol, which was ultrasonically dissolved, then added with 2.160 mL of sterilized water for injection, and mixed with vortex to obtain a solution of the dimaleate of the compound of formula (I) with a concentration of 2 mg/mL. | 2 | Prepared and used immediately |
| Toceranib phosphate | 40 | 12.19 mg of Toceranib phosphate was weighed into a preparation bottle, and added with approximately 0.240 mL of a mixed liquid of 5 vol% polyoxyethylene castor oil and 5 vol% anhydrous ethanol, which | 4 | Prepared and used immediately |

(continued)

| Compound | Dosage of administration (mg/kg) | Methods of preparation | Concentration (mg/mL) | Storage condition |
|---|---|---|---|---|
| | | was ultrasonically dissolved, then added with 2.160 mL of sterilized water for injection, and mixed with vortex to obtain a Toceranib phosphate solution with a concentration of 4 mg/mL. | | |
| Note: the medicine should be prepared and used immediately. Before administration, it is necessary to gently mix the medicine thoroughly. | | | | |

**Table 2. Grouping of experimental animals and administration protocols**

| Group | N[a] | Compound therapy | Dosage (mg/kg) | Administration volume parameter (μL/g)[b] | Administration route | Administration protocol |
|---|---|---|---|---|---|---|
| 1 | 8 | Vehicle | -- | 10 | PO | QD × 8d |
| 2 | 8 | Dimaleate of the compound of formula (I) | 20 | 10 | PO | QD ×8d |
| 3 | 8 | Toceranib phosphate solution | 40 | 10 | PO | QD × 8d |
| Note:<br>a. N: the number of mice in each group;<br>b. Administration volume: the drug was administered at a dose of 10 μL/g based on the body weight of the mice. If the body weight loss was > 15%, the administration was discontinued. If the body weight loss was ≤ 10%, the administration was resumed. | | | | | | |

The experimental indicators were to examine whether the tumor growth was inhibited, delayed or cured. The diameter of the tumor was measured with a vernier caliper. The calculation formula for tumor volume was: $V=0.5a\times b^2$, wherein a and b represented the long diameter and the short diameter of the tumor, respectively. The anti-tumor efficacy of the compound was determined by TGI (%). TGI (%) reflects the rate of tumor growth inhibition. Calculation of TGI (%): TGI (%) = [1-(Average tumor volume at the end of administration in a certain treatment group - Average tumor volume at the start of administration in this treatment group) / (Average tumor volume at the end of treatment in the vehicle control group - Average tumor volume at the start of treatment in the vehicle control group)] ×100%.

The experimental results are shown in Table 3.

**Table 3 Results of in vivo efficacy studies of compound (I) in mouse mast cell tumor P815 tumor model**

| Day | Tumor volume ($mm^3$)[a] | | |
|---|---|---|---|
| | Vehicle group | Compound of formula (I) group | Toceranib group |
| | | **20mg/kg** | **40mg/kg** |
| 0[b] | 100±4 | 100±4 | 100±4 |
| 2 | 142 ±7 | 141±22 | 127±6 |
| 4 | 289±26 | 244±35 | 231±14 |
| 6 | 423±42 | 319±55 | 299±15 |
| **8** | **825±50** | **483 ±93** | **510±38** |
| Note: a. Average value ±SEM; b. Days after administration. | | | |

Day 8 after administration, the average tumor volume of tumor-bearing mouse in the control vehicle group reached 825mm$^3$. Compared with the vehicle control group, the average tumor volumes of the test substance compound of formula (I) at a dosage of 20 mg/kg and Toceranib at a dosage of 40 mg/kg were 483 mm$^3$ (TGI = 47.25%, $p = 0.003$) and 510 mm$^3$ (TGI = 43.38%, $p < 0.001$), respectively.

The experimental results show that the compound of formula (I) of the present application achieves the same therapeutic effect when administered at half the dose of Toceranib, reduced therapeutically effective amount and reduced cost of medication, and helps to reduce the side effects of the drug with enhanced safety.

**Experimental Example 3: Evaluation of anti-tumor effects and adverse reactions of the compound of formula (I) of the present application against canine mast cell tumor**

Research method:

Since May 2021, a total of 14 dogs with mast cell tumors have been collected and enrolled for treatment with a dimaleate of the compound of formula (I).

Table 4: Clinical staging criteria for canine cutaneous mast cell tumors

| Staging | Characteristics |
|---|---|
| 0 | A single dermal mass. Postoperative histopathology confirmed that it was not completely resected and there was no lymph node metastasis.<br>a. Without a systemic symptom; b. With a systemic symptom |
| I | A single mass confined to the dermis layer without lymph node metastasis.<br>a. Without a systemic symptom; b. With a systemic symptom |
| II | A single mass confined to the dermis, accompanied by lymph node metastasis.<br>a. Without a systemic symptom; b. With a systemic symptom |
| III | Multiple dermal masses; large and infiltrative mass, with/without lymph node metastasis.<br>a. Without a systemic symptom; b. With a systemic symptom |
| IV | Distant metastasis occurs, including in the bone marrow or peripheral blood. |
| Note: derived from David M. Vail, Douglas H. Thamm, Julius M. Liptak. withrow & MacEwen's Small Animal Clinical Oncology 6th, ELSEVIER. 2019. | |

The affected dogs with mast cell tumor were administered with the drug of the dimaleate of the compound of formula (I) at a dose of 2.0 mg/kg to 3.25 mg/kg (all doses were calculated based on free base, the same below), orally once every other day. For affected dogs with assessable solid tumors, baseline assessment was performed before the first trial medication. After the administration of the first trial medication, mass measurement and blood routine examination were performed every 2 weeks, and blood biochemical examination and imaging examination were performed every 4 weeks to evaluate the response to tumor treatment until disease progression, intolerable drug withdrawal due to treatment-related side effects, or death of enrolled dogs, and to evaluate the efficacy and safety of the compound of formula (I) in the treatment of a canine mast cell tumor and a breast tumor. The primary observation endpoints: objective response rate (ORR), disease control rate (DCR), progression-free survival (PFS), and overall survival (OS); complete response (CR), partial response (PR), stable disease (SD), and progressive disease (PD).

**(1) A case of a mast cell tumor in a Golden Retriever**

A case of a Golden Retriever was presented to the hospital on September 26, 2021 for a mass at the tarsal joint of the right hind limb. Physical examination palpated the popliteal lymph node of the right hind limb was enlarged compared to the left side. Cytology of the mass was suggestive of a mast cell tumor. Blood routine, blood biochemistry, abdominal ultrasonography, and FNA of the liver and spleen were performed on October 3. No hepatic or splenic metastases were seen. The clinical staging was stage II. A sample of the affected dog was taken from the mass site and tested for exon 11 of the c-kit gene of the affected dog, and no mutation was seen. The baseline measurement value of the mass in the right hind limb was 2.5 ×2.2 cm (as shown in FIG. 1). The affected dog was treated with an dimaleate of the compound of formula (I) at an initial dose of 3.07 mg/kg started from October 3 for 14 days, and was then re-examined (on October 17). The measured value of the mass was 2.2×2.1 cm (as shown in FIG. 2), which decreased by 12% compared with the baseline. The tumor response was evaluated as stable disease (SD).

The affected dog was re-examined on November 1, and the measured value of the right hind limb mass was 2×1 cm (as shown in FIG. 3), with a significant reduction in thickness, and the tumor response was evaluated as partial remission (PR). Popliteal lymph node ultrasound suggested mild enlargement of the right popliteal lymph node (0.78 ×0.52 cm) compared to the left (0.68 ×0.29 cm). As the tumor has significantly shrunk, the owner is considering surgical removal. The affected dog underwent excision of right hind limb tarsal mass and removal of right hind limb popliteal lymph node on November 3. The pathology confirmed the diagnosis of mast cell tumor with popliteal lymph node metastasis, and the pathology was graded as grade II (three-way classification). Because the postoperative margins of the mass were assessed as deep marginal infiltration of tumor cells, the affected dog was continued being treated with dimaleate of the compound of formula (I) at a dose of 2.63 mg/kg started from December 5.

The affected dog was re-examined on postoperative days 47, 82, 117, and 145, respectively, and no recurrence of the mass was seen on the skin. As of March 28, 2022, the affected dog had been given 66 doses of medication, had a good mental and dietary appetite, and had a survival period of greater than 176 days.

**(2) A case of mast cell tumor in a Tibetan mastiff**

The affected dog was examined for a mass in the right hind limb and was diagnosed cytologically as a mast cell tumor with clinical stage II and no mutation was seen in c-kit.

On May 24, 2021, the dimaleate of the compound of formula (I) was administered at a dose of 2.84 mg/kg for treatment, and the baseline size of the mass was 7.1 ×4×4.5 cm. On June 21, the affected dog was re-examined, and the size of the mass was 6.6×3.9×4.4 cm. The affected dog was re-examined for blood routine and serum biochemistry, and no obvious abnormality was seen compared with the baseline. The treatment was continued at a dosage of 2.84 mg/kg. The affected dog was re-examined on July 19, and the size of the mass was 5.5 ×3.5 ×4.5 cm. Treatment was continued at a dose of 2.84 mg/kg. The affected dog died on September 18, 2021, and had a total survival period of 117 days.

**(3) A case of a mast cell tumor in a Labrador**

One year ago, it was consulted for a right axillary mass with cytology suggestive of mast cell tumor, and ultrasound suggestive of a large mass in the splenic head, and underwent removal of the right axillary mass and right axillary lymph node, as well as splenectomy. Pathology diagnosed the splenic mass as angiosarcoma and the axillary mass as mast cell tumor. The pathological grade of mast cell tumor was grade III (three-way classification). Doxorubicin chemotherapy was used 5 times 1 month after surgery.

The affected dog was revisited at the seventh postoperative month (December 13, 2021) for new masses on the back, and cephalic side of the vulva, all of which were cytologically diagnosed as mast cell tumors, with no other obvious metastatic foci, and clinical staging was stage III. Samples were taken from the tumor sites on the back and cephalic side of the vulva of the affected dog to detect exon 11 of the c-kit gene. The results showed that there was a 45bpITD mutation at the end of exon 11. The mass on the back was selected as the target lesion, and the measured value of the baseline was 1.5 cm.

The affected dog was treated with the dimaleate of the compound of formula (I) at an initial dose of 2.42 mg/kg started from December 13, and after 14 days of treatment, it came to the hospital for re-examination on December 26. The tumor on the back disappeared and could not be measured. The tumor response was evaluated as CR. The mass on the cephalic side of the vulva was significantly reduced.

The affected dogs came to the hospital for re-examination on day 42 and day 77 of treatment, respectively. No recurrence of the mass on the back was observed in either case, and the tumor response was evaluated as CR. No enlargement of the mass on the cephalic side of the vulva was observed.

The affected dog came to the hospital for re-examination on day 126 of treatment. There was no recurrence of the mass on the back. The mass on the cephalic side of the vulva was red, swollen and enlarged, with a measured value of 1.74 ×1.9 cm. The tumor response of the target lesion was evaluated as CR, and the response of the non-target lesion was evaluated as PD.

The affected dog is still undergoing treatment up to now. No recurrence of the mass on the back has been observed, and no new lesions have been found. The overall efficacy assessment of the target lesion is CR.

As of April 17, 2022, the affected dog had received medication 51 times. The affected dog had a good mental and a good dietary desire, and had a survival period of greater than 126 days.

**(4) A case of a mast cell tumor in a Golden Retriever**

The affected dog was presented to the hospital on January 2, 2022 for a nasal skin mass and was diagnosed cytologically as a mast cell tumor with clinical stage I, and no mutations were seen in the c-kit. The dimaleate of the compound of formula (I) was given at a dose of 2.4 mg/kg and no significant adverse effects were observed after

administration. The affected dog was re-examined on February 13. The blood routine and serum biochemistry were re-examined, and no obvious abnormalities were found. The treatment was continued at a dose of 2.4mg/kg. After taking the medicine, symptoms of soft stool and diarrhea occurred. The affected dog was re-examined on April 10. The blood routine and serum biochemistry were re-examined, and no obvious abnormalities were found. The treatment was continued at a dose of 2.3 mg/kg, and no obvious adverse reactions occurred after medication. The affected dog was re-examined on June 6. Nasal ultrasound suggests that the subcutaneous mass is suspected of invading the nasal cavity. The blood routine and serum biochemistry were re-examined, and no obvious abnormalities were found. The treatment was continued at a dose of 2.3 mg/kg, and no obvious adverse reactions occurred after medication. The affected dog was re-examined on August 7. Nasal ultrasound suggests a subcutaneous mass accompanied by invasive changes in adjacent turbinate bone. The blood routine was re-examined, and no obvious abnormalities were found. Treatment was continued at a dose of 2.4 mg/kg. The affected dog is still undergoing drug therapy with the dimaleate of the compound of formula (I) and has a survival period of greater than 259 days.

**(5) A case of a mast cell tumor in a Golden Retriever**

The affected dog was diagnosed cytologically as a mast cell tumor due to a skin mass on the left hind limb, and the measured value of the baseline for the hind limb mass was 3.5 ×3.0 cm clinical stage III. On September 3, 2022, the dimaleate of the compound of formula (I) was administered at a dose of 3.25 mg/kg, and soft stools appeared once after dosing. The dosage was adjusted to 2.75 mg/kg on September 7 to continue treatment, and there were no significant adverse reactions after dosing. On October 15, the measured value of the hind limb mass of the affected dog was 3.0×2.9 cm. Thin and watery stools occurred during a period. The treatment was adjusted to continue at a dose of 2.25 mg/kg, and no obvious adverse reactions occurred after medication. On November 6, the measured value of the hind limb mass of the affected dog was 2.3 ×2.5 cm. The affected dog is still under continuous treatment.

The above results of animal clinical trials show that the compound of formula (I) and the salt thereof of the present application can effectively inhibit the growth of mast cell tumors and prolong the survival period of the animals.

**(6) Occurrence of adverse reactions**

Treatment-related clinical adverse reactions in this experiment were all grades 1 to 2, including diarrhea (n=9, 64.3%), blood in stool (n=4, 28.6%), decreased appetite (n=4, 28.6%), vomiting (n=3, 21.4%), and occasionally blood in urine (n=1, 7.1%). No grade 3 or 4 adverse reactions were observed. (Adverse event assessment criteria refer to VCOG-CTCAE (Veterinary cooperative oncology group-common terminology criteria for adverse events (VCOG-CTCAE) following chemotherapy or biological antineoplastic therapy in dogs and cats v1.1. Vet Comp Oncol. 2016)).

Furthermore, according to the instruction manual for Toceranib phosphate tablets Palladia® providing summary information on adverse reactions, in which diarrhea of all grades accounted for 46% of the total, with 6.9% of grade 3 or 4 adverse reactions; anorexia of all grades accounted for 39.1% of the total, with 6.9% of grade 3 or 4 adverse reactions; and vomiting of all grades accounted for 32.2% of the total, with 9.2% of grade 3 or 4 adverse reactions. Hemorrhage in stool/gastrointestinal bleeding/hemorrhagic diarrhea of all grades accounted for 12.6% of the total, with grade 3 or 4 adverse reactions accounting for 2.3%.

From the above results, it can be seen that the compounds of formula (I) and salts thereof showed grade 1-2 adverse reactions in the gastrointestinal tract, whereas Toceranib showed grade 3 to 4 adverse reactions, indicating that the compounds of formula (I) and salts thereof have a much lower level of adverse reactions and have a superior safety.

The above description is merely the preferred embodiments of the present application and is not intended to limit the present application. Any modifications, equivalent replacements, improvements, etc. made within the spirit and principles of the present application should all be included within the scope of protection of the present application.

## Claims

1. Use of a compound N-(5-((Z)-(5-fluoro-2-carbonylindole-3-ylidene)methyl)-2,4-dimethyl-1H-pyrrol-3-yl)-3-(4-methylpiperazin-1-yl) propanamide represented by formula (I), or a pharmaceutically acceptable salt thereof in the manufacture of a medicament for treatment of a mast cell tumor in a non-human mammal,

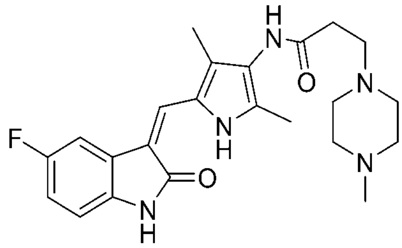

formula (I).

2. The use according to claim 1, wherein the mast cell tumor includes a cutaneous mast cell tumor and a systemic mast cell tumor.

3. The use according to claim 1 or 2, wherein the mast cell tumor is a cutaneous mast cell tumor.

4. The use according to any one of claims 1-3, wherein the mast cell tumor is a recurrent mast cell tumor.

5. The use according to any one of claims 1-4, wherein the mast cell tumor is an unresectable grade 2 or grade 3 recurrent cutaneous mast cell tumor.

6. The use according to any one of claims 1-5, wherein the mast cell tumor is a tumor caused by a mutation of c-kit gene.

7. The use according to any one of claims 1-6, wherein the compound of formula (I) or the pharmaceutically acceptable salt thereof is administered to the non-human mammal at least once daily or every other day, at a dose of 0.1 mg/kg to 10 mg/kg.

8. The use according to any one of claims 1-7, wherein the compound of formula (I) or the pharmaceutically acceptable salt thereof is administered to the non-human mammal at least once daily or every other day, at a dose of 1 mg/kg to 5 mg/kg.

9. The use according to any one of claims 1-8, wherein the compound of formula (I) or the pharmaceutically acceptable salt thereof is administered to the non-human mammal at least once daily or every other day, at a dose of 1.5 mg/kg to 3.5 mg/kg.

10. The use according to any one of claims 1-9, wherein the compound of formula (I) or the pharmaceutically acceptable salt thereof is administered to the non-human mammal at least once daily or every other day, at a dose of 2 mg/kg to 3.5 mg/kg.

11. The use according to any one of claims 1-10, the compound of formula (I) or the pharmaceutically acceptable salt thereof can be administered to the non-human mammal via any of routes of oral administration, parenteral administration, rectal administration, cutaneous administration, nasal or inhalation administration, and intra-tumoral administration.

12. The use according to any one of claims 1-11, wherein the pharmaceutically acceptable salt of the compound of formula (I) is a dimaleate.

13. The use according to any one of claims 1-12, wherein the non-human mammal is selected from the group consisting of canine, feline, murine and equine.

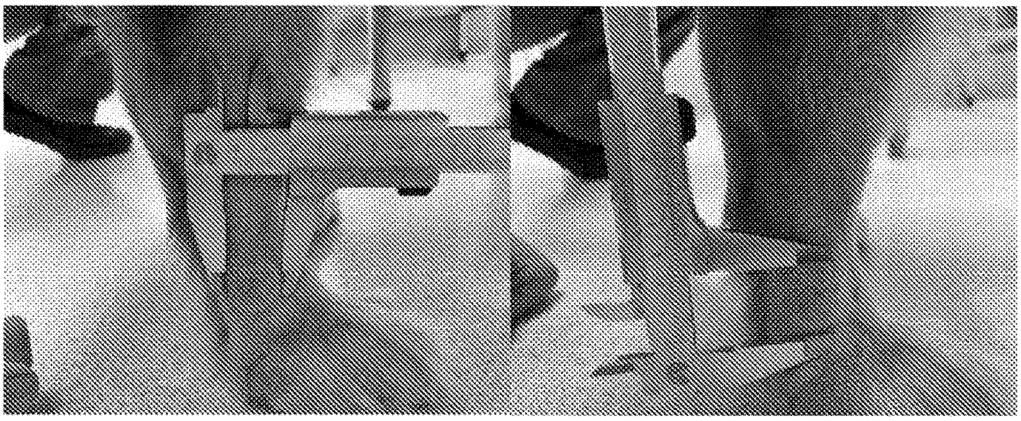

FIG. 1

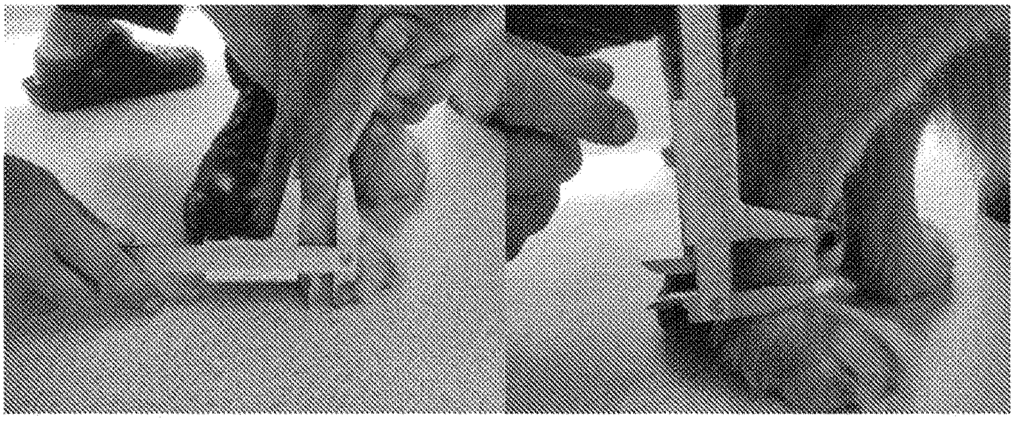

FIG. 2

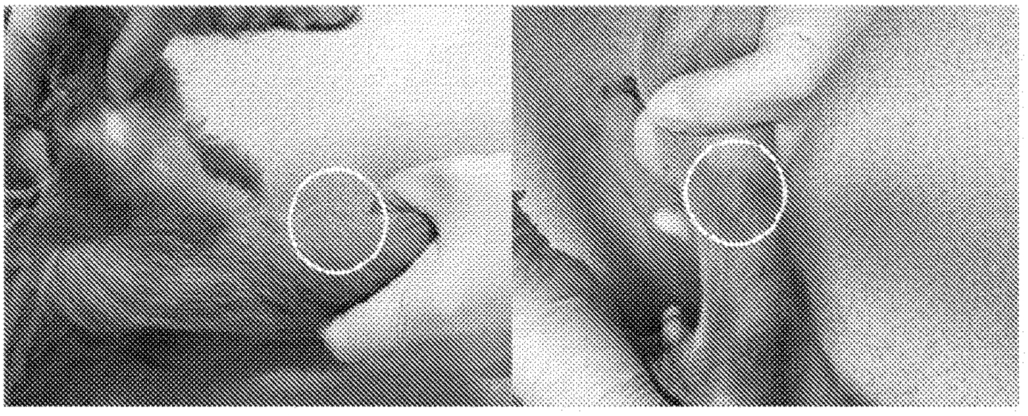

FIG. 3

**INTERNATIONAL SEARCH REPORT**

International application No.
**PCT/CN2024/076530**

**A. CLASSIFICATION OF SUBJECT MATTER**

A61K31/496(2006.01)i; A61K31/4439(2006.01)i; A61K31/404(2006.01)i; C07D403/06(2006.01)i; C07D401/14(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K C07D A61P

Documentation searched other than minimum documentation to the extent that such documents are included in the fields searched

Electronic data base consulted during the international search (name of data base and, where practicable, search terms used)

CNTXT; ENTXTC; VCN; CJFD; DWPI; CNKI; WFNPL; NPTXT; NPABS; Web of Science; STNext; 读秀, DUXIU: N-(5-((Z)-(5-氟-2-羰基吲哚-3-亚)甲基)-2, 4-二甲基-1氢-吡咯-3-基)-3-(4-甲基哌嗪-1-基)丙酰胺, 肥大细胞瘤, 式(1)structural formula search, c-kit, Mastocytoma, Propionamide, Methylpiperazine, Carbonylindole, P815

**C. DOCUMENTS CONSIDERED TO BE RELEVANT**

| Category* | Citation of document, with indication, where appropriate, of the relevant passages | Relevant to claim No. |
|---|---|---|
| A | CN 105566302 A (QILU PHARMACEUTICAL CO., LTD.) 11 May 2016 (2016-05-11)<br>description, paragraphs [0005], [0127], and [0128] | 1-13 |
| A | CN 104119321 A (QILU PHARMACEUTICAL CO., LTD.) 29 October 2014 (2014-10-29)<br>claims 1-13 | 1-13 |
| A | CN 113999221 A (NANJING UNIVERSITY OF CHINESE MEDICINE) 01 February 2022 (2022-02-01)<br>claims 1-10 | 1-13 |
| A | WO 2011153814 A1 (QILU PHARMACEUTICAL CO., LTD. et al.) 15 December 2011 (2011-12-15)<br>claims 1-10 | 1-13 |
| A | 李格宾等 (LI, Gebin et al.). "应用分子靶点药物治疗犬肥大细胞瘤的研究进展 (Progress on Application of Molecular Target Drugs in Treatments of Canine Mast Cell Tumors)" *动物医学进展 (Progress in Veterinary Medicine),* Vol. 37, No. 2, 31 December 2016 (2016-12-31), pages 80-84<br>abstract | 1-13 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 May 2024** | **13 May 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/076530**

| Patent document cited in search report | | | Publication date (day/month/year) | Patent family member(s) | | | Publication date (day/month/year) |
|---|---|---|---|---|---|---|---|
| CN | 105566302 | A | 11 May 2016 | None | | | |
| CN | 104119321 | A | 29 October 2014 | WO | 2014177011 | A1 | 06 November 2014 |
| CN | 113999221 | A | 01 February 2022 | None | | | |
| WO | 2011153814 | A1 | 15 December 2011 | CA | 2801939 | A1 | 15 December 2011 |
| | | | | CA | 2801939 | C | 24 November 2015 |
| | | | | US | 2013158030 | A1 | 20 June 2013 |
| | | | | US | 8637681 | B2 | 28 January 2014 |
| | | | | KR | 20130092541 | A | 20 August 2013 |
| | | | | KR | 101760651 | B1 | 24 July 2017 |
| | | | | JP | 2013529220 | A | 18 July 2013 |
| | | | | JP | 5938400 | B2 | 22 June 2016 |
| | | | | EP | 2581371 | A1 | 17 April 2013 |
| | | | | EP | 2581371 | A4 | 30 October 2013 |
| | | | | EP | 2581371 | B1 | 07 June 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202310100761
- WO 2011153814 A1
- CN 104119321 A

### Non-patent literature cited in the description


- **S. M. BERGE et al.** *J. Pharmaceutical Sciences*, 1977, vol. 66, 1
- **DAVID M. VAIL** ; **DOUGLAS H. THAMM** ; **JULIUS M. LIPTAK**. withrow & MacEwen's Small Animal Clinical Oncology. ELSEVIER, 2019